(19) Europäisches Patentamt
European Patent Office
Office européen des brevets

(11) EP 1 301 460 B1

(12) **FASCICULE DE BREVET EUROPEEN**

(45) Date de publication et mention de la délivrance du brevet:
**06.08.2008 Bulletin 2008/32**

(21) Numéro de dépôt: **01956605.8**

(22) Date de dépôt: **18.07.2001**

(51) Int Cl.:
***C07C 69/52*** (2006.01) ***A23L 1/30*** (2006.01)

(86) Numéro de dépôt international:
**PCT/FR2001/002340**

(87) Numéro de publication internationale:
**WO 2002/006205 (24.01.2002 Gazette 2002/04)**

(54) **PROCEDE DE PREPARATION D'UN ESTER DE CORPS GRAS ET SON UTILISATION DANS LES DOMAINES PHARMACEUTIQUE, COSMETIQUE OU ALIMENTAIRE**

VERFAHREN ZUM HERSTELLEN VON FETTSÄUREESTERN UND IHRE VERWENDUNG IN ARZNEIMITTELN, KOSMETIKA UND NAHRUNGSMITTELN

METHOD FOR PREPARING A FATTY SUBSTANCE ESTER AND USE THEREOF IN PHARMACEUTICS, COSMETICS OR FOOD INDUSTRY

(84) Etats contractants désignés:
**AT BE CH CY DE DK ES FI FR GB GR IE IT LI LU MC NL PT SE TR**

(30) Priorité: **19.07.2000 FR 0009506**

(43) Date de publication de la demande:
**16.04.2003 Bulletin 2003/16**

(73) Titulaire: **Laboratoires Expanscience**
**92419 Courbevoie Cedex (FR)**

(72) Inventeurs:
- **BARRAULT, Joel**
  **F-86240 Liguge (FR)**
- **BOISSEAU, Mickael**
  **F-86170 Blaslay (FR)**
- **POUILLOUX, Yannick**
  **F-86550 Mignoloux-Beauvoir (FR)**
- **PICCIRILLI, Antoine**
  **F-28230 Epernon (FR)**

(74) Mandataire: **Callon de Lamarck, Jean-Robert**
**Cabinet Régimbeau**
**20, rue de Chazelles**
**75847 Paris cedex 17 (FR)**

(56) Documents cités:

| | |
|---|---|
| **EP-A- 0 585 071** | **US-A- 4 393 044** |
| **US-A- 4 748 161** | **US-A- 5 502 045** |

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un nouveau procédé de préparation d'un ester de corps gras et d'un alcool choisi dans le groupe constitué par les stérols, les stanols, les 4-méthylstérols et leurs homologues hydrogénés, les alcools triterpéniques et leurs homologues hydrogénés, et les mélanges de ceux-ci, cet ester étant en particulier destiné à un usage pharmaceutique, notamment dermatologique, ainsi qu'à un usage cosmétique ou alimentaire.

Les phytostérols (famille des phytohormones) et les acides gras essentiels sont des composés à forte activité biologique et intéressent à ce titre les domaines de la pharmacie, de la cosmétique et de l'alimentation humaine.

Les phytostérols sont des composés d'origine terpénique qui constituent la fraction majoritaire des insaponifiables végétaux, à l'instar du β-sitostérol. Depuis les années 50, les phytostérols sont notamment connus pour leur action hypocholestérolémiante (Ling & Jones (1995), Life Sciences, Vol. 57, n°3, pp. 195-206). L'hypothèse du mécanisme d'action est la suivante : les phytostérols provoquent une diminution du cholestérol sanguin par compétition avec ce dernier pour sa solubilisation au sein des micelles des sels biliaires dans l'intestin. En outre, les stérols entraînent une diminution du cholestérol sanguin (LDL) et une légère augmentation de la synthèse et de l'excrétion du cholestérol endogène.

De façon analogue, les acides gras polyinsaturés (AGPI) jouent un rôle essentiel sur le plan nutritionnel. A titre d'exemple, l'acide linoléique est un composé indispensable à l'organisme puisque ce dernier ne peut le synthétiser (Delplanque et all. (1999), Oléagineux Corps Gras Lipides, Vol. 1, pp. 86-93). Il est en effet le chef de file de la série métabolique des AGPI dits n-6 ou ω-6, acides gras vitaux pour l'organisme.

Cette série d'acides comprend notamment l'acide arachidonique (C20 :4) qui est à la base de la synthèse de médiateurs chimiques tels que les eïcosanoides qui contrôlent de nombreuses fonctions de l'organisme, notamment l'agrégation plaquettaire, la fonction rénale et la réponse immunitaire. Parmi les acides gras essentiels, on peut aussi citer les acides eicosapentaenoique (EPA) et docosahexaenoique (DHA) pour leur rôle préventif des maladies cardiovasculaires et de certains cancers.

Enfin, sur le plan dermo-cosmétique, les stérols et les acides gras essentiels sont aussi des composés incontournables. Les stérols sont connus pour leurs propriétés anti-inflammatoires et anti-érythémateuse, ainsi que pour leur action apaisante et restructurante (Wachter, Salka, Magnet, Cosmetics & Toileteries, (1995), Vol. 110, pp 72-80). Par ailleurs, en limitant les pertes d'eau trans-épidermales, les acides gras mono et polyinsaturés ont quant à eux une action hydratante et nutritive. Stérols et AGPI jouent enfin un rôle important dans la synthèse des lipides de la barrière cutanée épidermique.

Dans le but d'accroître la biodisponibilité des stérols, et de leur homologues saturés les stanols, il est connu dans l'art antérieur d'augmenter la lipophylie de ces composés en les soumettant à une réaction d'estérification avec un corps gras, notamment un acide gras ou un dérivé de ce dernier, de préférence en présence d'un catalyseur.

Actuellement, les stérols et stanols sont estérifiés par réaction de transestérification en présence de catalyseurs basiques homogènes tels que le méthylate de sodium. La présence de NaOMe nécessite des étapes supplémentaires de destruction du catalyseur qui conduisent à la formation de sels qu'il faut impérativement détruire. Toutes ces dernières opérations représentent un coût supplémentaire très important dans la production de ces esters de stérols et de stanols. De plus, l'utilisation de catalyseurs homogènes peut favoriser les réactions secondaires de dégradation des composés pouvant modifier l'aspect final du produit (couleur et/ou odeur).

US 5 502 045 décrit ainsi une réaction d'estérification d'un stanol par transestérification en présence d'un catalyseur basique homogène, tel que l'éthylate de sodium.

La catalyse hétérogène, par rapport à la catalyse homogène, présente l'avantage de mettre en oeuvre des catalyseurs plus facilement séparables du milieu réactionnel et de susciter moins de problèmes de corrosion et de réactions secondaires. On peut ainsi citer l'oxyde de magnésium qui toutefois présente l'inconvénient de provoquer des réactions secondaires de déshydratation et/ou des réactions d'isomérisation des liaisons insaturées, rédhibitoires notamment dans le cas de produits destinés à l'alimentation.

EP 585 071 décrit l'estérification d'acide gras avec au moins un sel d'acide hydroxyalcanesulfonique à l'aide d'un catalyseur du type catalyseur hétérogène, tel que des oxydes métalliques, par exemple l'oxyde de magnésium.

On a maintenant trouvé de manière tout à fait surprenante et inattendue que l'utilisation d'une certaine classe de composés permet d'obtenir un excellent effet de catalyse hétérogène pour la réaction d'estérification mettant en jeu au moins un corps gras et au moins un stérol et/ou un stanol et/ou un 4-méthylstérol ou un homologue hydrogéné de ce dernier et/ou un alcool triterpénique ou un homologue hydrogéné de ce dernier. Les esters de corps gras obtenus par le procédé selon l'invention sont tout particulièrement appropriés aux secteurs de la pharmacie, notamment de la dermatologie, de la cosmétique et de l'alimentation particulière (aliments fonctionnels, alicaments, cosmetofood).

La présente invention se rapporte ainsi à un procédé de préparation d'un ester de corps gras, caractérisé en ce que l'on soumet à une réaction d'estérification au moins un corps gras avec au moins un composé alcool choisi dans

le groupe constitué par les stérols, les stanols, les 4-méthylstérols et leurs homologues hydrogénés, les alcools triterpéniques et leurs homologues hydrogénés, et les mélanges de ceux-ci, en présence d'au moins un catalyseur solide choisi dans le groupe constitué par les oxydes de lanthanides et les mélanges de ces oxydes.

Par "catalyseur solide", on entend selon l'invention un catalyseur non dissous dans le reste du milieu réactionnel liquide et recyclable après usage.

Par "oxyde de lanthanide", on entend selon l'invention un oxyde choisi dans le groupe constitué par les oxyde de lanthane, les oxydes de cérium, les oxydes de praséodyme, les oxydes de néodyme, les oxydes de prométhium, les oxydes de samarium, les oxydes d'europium, les oxydes de gadolinium, les oxydes de terbium, les oxydes de dysprosium, les oxydes d'holmium, les oxydes d'erbium, les oxydes de thulium, les oxydes d'ytterbium, les oxydes de luténium et les mélanges de ces oxydes.

De préférence, le catalyseur solide est choisi dans le groupe constitué par l'oxyde de lanthane $La_2O_3$, l'oxyde cérique $CeO_2$, les oxydes de praséodyme $PrO_2$, $Pr_6O_{11}$ et $Pr_2O_3$, l'oxyde de samarium $Sm_2O_3$ et les mélanges de ces oxydes.

Plus particulièrement, le catalyseur solide est de préférence l'oxyde de lanthane $La_2O_3$.

Le catalyseur solide peut se présenter en particulier sous une forme solide choisie dans le groupe constitué par les poudres, les grains (pellets), les billes, les formes extrudées et les mélanges de celles-ci.

De préférence, le catalyseur solide est sous la forme d'une poudre avec une taille moyenne de particules comprise en particulier entre environ 1 et environ 1000 micromètres, plus particulièrement entre environ 10 et environ 500 micromètres et tout particulièrement de préférence entre environ 50 et environ 100 micromètres.

Le catalyseur solide peut être supporté sur un support inerte, c'est à dire un support inerte en présence des réactifs et produits de la réaction d'estérification, tels que les supports inertes poreux ou non poreux connus de l'homme du métier, par exemple l'alumine ou la silice.

Les oxydes de lanthanides, utilisés en tant que catalyseur dans le procédé selon l'invention, peuvent être préparés selon des méthodes connues de l'homme du métier, y compris lorsqu'il s'agit d'un catalyseur supporté. Ils sont également disponibles dans le commerce, tel que les "LSA" et "HSA" commercialisés par la société Rhodia.

La masse de catalyseur à utiliser dans le procédé selon l'invention peut être déterminée par l'homme du métier utilisant ses connaissances générales notamment sur la réaction d'estérification.

En particulier, le catalyseur solide utilisé dans le procédé selon l'invention est présent dans le milieu réactionnel en une proportion comprise entre environ 0,01 et environ 30 % en poids, et plus particulièrement entre 1 et 5 % en poids, par rapport au poids total du milieu réactionnel.

La réaction d'estérification mise en oeuvre dans le procédé selon l'invention peut être toute réaction d'estérification dans laquelle un des réactifs de départ est un stérol, et/ou un stanol et/ou un 4-méthylstérol ou un homologue hydrogéné de ce dernier et/ou un alcool triterpénique ou un homologue hydrogéné de ce dernier, telle que la réaction d'estérification directe entre ces composés alcools et un acide carboxylique ou son chlorure d'acide ou anhydride, ou encore la réaction de transestérification par alcoolyse entre ces composés alcools et un ester.

Par "corps gras", on entend selon l'invention, en accord avec les connaissances générales de l'homme du métier, une molécule comprenant, d'une part, au moins une fonction chimique pouvant réagir avec un composé alcool dans une réaction d'estérification comme décrite ci-dessus, notamment une fonction acide carboxylique, chlorure d'acide, anhydride d'acide ou encore une fonction ester dans le cas d'une transestérification par alcoolyse. D'autre part, la molécule du corps gras comprend au moins une chaîne hydrocarbonée "grasse" c'est-à-dire une chaîne hydrocarbonée linéaire d'au moins 7 atomes de carbone, insaturée ou non insaturée, éventuellement substituée, en particulier une chaîne hydrocarbonée linéaire en $C_7$-$C_{30}$. La chaîne grasse est de préférence une chaîne hydrocarbonée linéaire en $C_7$-$C_{30}$ éthyléniquement insaturée, comprenant au moins une insaturation éthylénique. Plus particulièrement, la chaîne grasse est une chaîne hydrocarbonée linéaire en $C_7$-$C_{30}$ éthyléniquement insaturée, comprenant au moins deux insaturations éthyléniques, conjuguées ou non conjuguées, comme par exemple dans le cas du linoléate de méthyle.

En particulier, le corps gras est choisi dans le groupe constitué par les acides gras saturés notamment l'acide caprique, l'acide laurique, l'acide stéarique, ou encore l'acide myristique, l'acide palmitique et les mélanges de ceux-ci, et les acides gras insaturés notamment l'acide undécylénique, l'acide oléique, l'acide ricinoléique, l'acide linoléique, ou encore les acides oméga-3 tels que l'acide linolénique et ceux de formules:

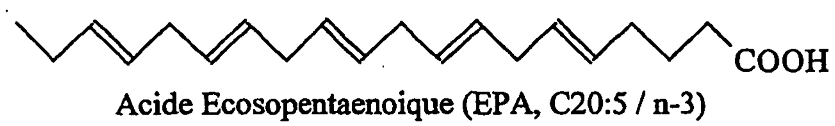

Acide Ecosopentaenoique (EPA, C20:5 / n-3)

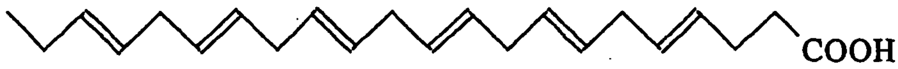

Acide Docosohexaenoique (DHA, C22:6 / n-3)

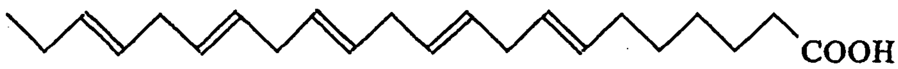

Acide Docosopentaenoique (DPA, C22:5 / n-3)

et les mélanges de ceux-ci.

Parmi les acides gras utilisables comme corps gras pour ces réactions d'estérification, on citera également en particulier les acides gras obtenus à partir des savons d'acide gras qui sont des sous-produits de la saponification d'une huile végétale ou du raffinage des huiles (soap stocks). Il s'agit en effet d'une valorisation très intéressante de ces sous-produits de la préparation des insaponifiables d'huile végétale.

Parmi les huiles végétales pouvant être utilisées on peut citer en particulier l'huile de tournesol, de palme, de palmiste, de noix de coco, de pépin de raisin, de moutarde noire, d'ocillette, de beurre de karité, d'amande douce, de soja, d'avocat, de lupin , d'arachide, de coton, de sésame, d'olive, de maïs, de cacao, de ricin, de Ben, de lin, de colza, de rocouyer, de germe de blé, de carthame, de noix, de noisette, de navette, de l'huile de son de riz et les mélanges de ces huiles.

La saponification de l'huile, notamment de l'huile d'avocat (ou de soja) est une étape essentielle du procédé d'obtention des insaponifiables. Cette étape, réalisée en présence de potasse aqueuse et d'éthanol, est une hydrolyse basique de l'huile (triglycérides) conduisant à la formation de savons de potassium et de glycérol :

$$(RCO_2)CH_2CH(O_2CR)CH_2(O_2CR) \quad + \quad 3\ KOH \longrightarrow 3\ RCOO^-K^+ \quad + \quad HOCH_2CH(OH)CH_2OH$$

| Triglycérides | Potasse | Savons | Glycérol |
|---|---|---|---|

L'insaponifiable, en émulsion dans la phase hydro-alcoolique (phase "savonneuse"), est ensuite extrait par le dichloroéthane (DCE) selon un procédé d'extraction liquide-liquide.

Après l'étape d'extraction liquide-liquide, la phase "savonneuse" est acidifiée par l'acide sulfurique. Les savons sont alors transformés en acides gras (réaction 1 ci-dessous). Le mélange obtenu est ensuite distillé afin d'éliminer l'éthanol et les traces de DCE. Les acides gras et l'eau sont enfin séparés par décantation.

$$2\ RCOO^-K^+ + H_2SO_4 \rightarrow 2\ RCOOH + K_2SO_4 \qquad (1)$$

Ces acides gras bruts d'avocat sont enfin purifiés, par exemple sur une colonne de silice (éluant hexane puis hexane-éther diéthylique 95:5) ou par distillation moléculaire et peuvent ainsi constituer la matière première mise en oeuvre lors de la préparation d'esters gras d'avocat et de stérol, et/ou de stanol ou d'un homologue hydrogéné de ce dernier et /ou d'alcool triterpénique ou d'un homologue hydrogéné de ce dernier, selon le procédé de la présente invention.

Les acides gras de soja ou d'une autre huile végétale telle que celles citées ci-dessus peuvent être obtenus selon la même voie de synthèse.

Ainsi, selon un mode de réalisation particulier, le corps gras estérifié selon l'invention est un acide gras d'au moins une huile végétale hydrogénée ou non, étant entendu que l'expression "acide gras d'huile végétale" englobe selon l'invention les acides gras présents à l'origine dans ladite huile végétale et les acides gras pouvant être obtenus par traitement de la phase savonneuse après saponification de ladite huile végétale, comme décrit ci-dessus.

Enfin, le corps gras estérifié selon l'invention, dans le cas d'une estérification par alcoolyse (transestérification), peut être un ester d'un acide gras tel que les acides gras décrits ci-dessus, en particulier un ester dont la partie alkyle du groupe alcoxy est une partie alkyle en $C_1$-$C_{22}$, par exemple une partie éthylhexyle, et plus particulièrement une partie alkyle en $C_1$-$C_6$ tel que le groupe méthyle, éthyle, propyle, isopropyle, butyle, tertiobutyle, pentyle ou hexyle, et plus particulièrement encore une partie alkyle en $C_1$-$C_3$. On peut citer notamment le laurate de méthyle, le myristate de méthyle ou encore l'oléate de méthyle, le linoléate de méthyle, le stéarate de méthyle, l'undécylénate de méthyle, l'oléate de butyle, le ricinoléate de méthyle, le palmitate de méthyle ou encore le palmitoélate de méthyle et les mélanges de

ceux-ci..

Avantageusement, le corps gras estérifié selon l'invention peut être sous la forme d'au moins une huile végétale hydrogénée ou non, contenant des acides gras et/ou des esters d'acides gras, telle que les huiles végétales déjà citées ci-dessus ou un mélange de ces huiles hydrogénées ou non.

Le composé alcool utilisé dans le procédé selon l'invention est choisi dans le groupe constitué par les stérols, les stanols, les 4-méthylstérols et leurs homologues hydrogénés, les alcools triterpéniques et leurs homologues hydrogénés, et les mélanges de ceux-ci.

Les stérols et stanols utilisés en tant que produit de départ dans le procédé selon l'invention sont des composés bien connus de l'homme du métier.

Par "stérol", on entend plus particulièrement selon l'invention le stérol, c'est à dire le composé perhydro-1,2-cyclopentanophenanthrène ayant un groupe hydroxyle à la position 3, et les analogues du stérol de formule générale (I) ci-dessous.

Par "stanol", on entend selon l'invention le dérivé hydrogéné à la position 5 d'un stérol donné tel que défini ci-dessus.

Ainsi, de préférence, les stérols et stanols utilisables comme produits de départ dans le procédé selon l'invention répondent à la formule générale suivante :

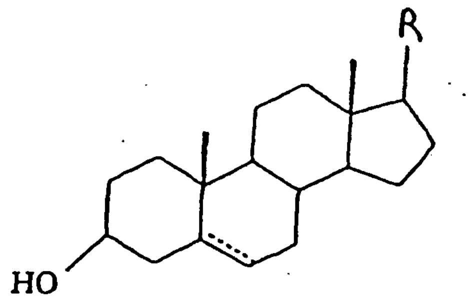

(I)

dans laquelle l'insaturation en pointillés en position 5 correspond à l'insaturation dans le cas des stérols, R représente une chaîne hydrocarbonée, linéaire ou ramifiée, insaturée ou non, comportant de 1 à 25 atomes de carbone. En particulier, R est choisi dans le groupe constitué par les groupes alkyle en $C_1$-$C_{12}$ les groupe alcoxy en $C_1$-$C_8$, les groupes alcényles en $C_2$-$C_8$, les groupe alcynyles en $C_2$-$C_8$, les groupes cycloalkyle en $C_3$-$C_8$, les groupes alcényles en $C_2$-$C_8$ halogénés, les groupes alcynyles en $C_2$-$C_8$ halogénés. Le terme "halogéné" désigne un ou plusieurs substituant halogène, à savoir un ou plusieurs atome(s) de chlore, fluor, brome ou iode.

Parmi les stérols pouvant être avantageusement utilisés dans le procédé selon l'invention, on peut citer en particulier le β-sitostérol, l'α-sitostérol, le γ-sitostérol, le stigmastérol, ou encore le campestérol et les mélanges de ceux-ci. Par exemple, le β-sitostérol peut être utilisé sous la forme du produit dénommé "Ultra" (comprenant principalement du β-sitostérol) tel que commercialisé par la société Kaukas. Dans le cas d'une utilisation d'un mélange de stérols, on peut citer par exemple le produit dénommé "Generol" comprenant principalement du β-sitostérol (environ 50 % en poids), du stigmastérol et du campestérol tel que commercialisé par la société Henkel ou encore le produit "Primal" de la société Kaukas.

Parmi les stanols pouvant être avantageusement utilisés dans le procédé selon l'invention, on peut citer en particulier le β-sitostanol, le stigmastanol, ou encore le campestanol et les mélanges de ceux-ci. Bien entendu, comme cela est bien connu de l'homme du métier, les stanols utilisés dans le procédé selon l'invention peuvent être obtenus par hydrogénation catalytique des stérols, tels que les stérols cités ci-dessus, par exemple lors d'une étape en amont de l'étape d'estérification selon l'invention, un utilisant des catalyseurs bien connus comme le palladium, le platine, le cuivre ou encore le nickel.

Les 4-méthylstérols, comme décrits en particulier dans le "Manuel des Corps Gras", publié aux éditions Tec&Doc Lavoisier sous l'égide de l'Association Française pour l'Etude des Corps Gras (édition 1992, Alain Karleskind et Jean-Pierre Wolff), ont pour origine biosynthétique le cycloarténol et le 24-méthylènecycloartanol chez les végétaux, le lanostérol chez les animaux : ils sont caractérisés par la présence d'un seul groupe méthyle en 4a, l'autre ayant subi une dégradation oxydative et une élimination sous forme de dioxyde de carbone (BENVENISTE, 1986). Le groupe méthyle en 14a peut soit subsister, soit être éliminé sous forme d'acide formique et remplacé par un atome d'hydrogène, le noyau cyclopropanique en 9-10-19 peut être maintenu, ou bien subir une ouverture, donnant naissance au groupe méthyle 19 et à une double liaison localisée en 9(11) ou en 8(9) . La nomenclature usuelle utilise le nom de l'alcool

triperpénique voisin précédé de "31-nor"; la nomenclature systématique est basée sur le nom du stérol voisin, précédé de "4α-méthyl".

Les 4-méthylstérols pouvant être utilisés comme composé alcool dans le procédé selon l'invention sont en particulier ceux de formule (II) suivante :

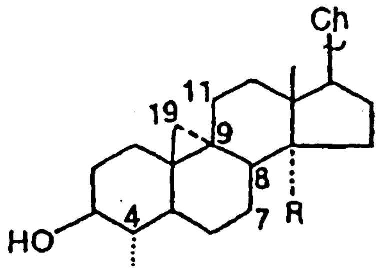

dans laquelle le substituant Ch représente un groupe ayant l'une des formules a à h suivantes :

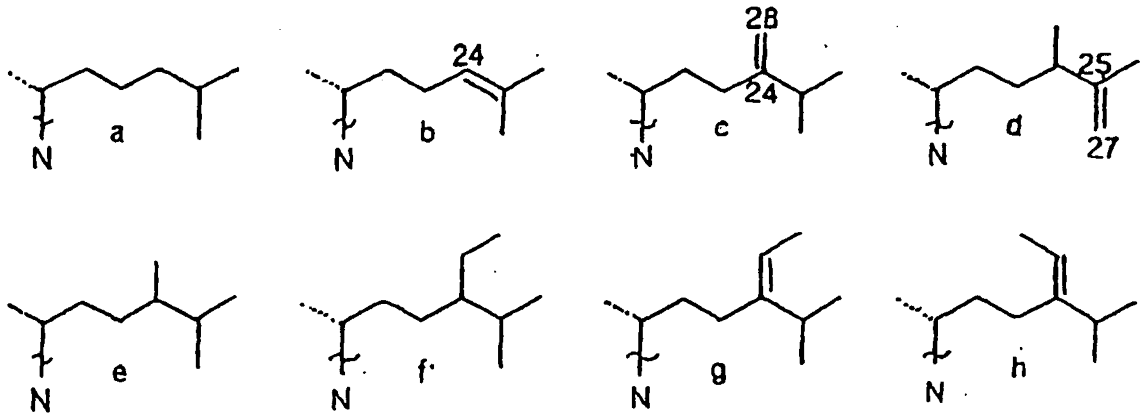

Le tableau 1 suivant cite des 4-méthylstérols répondant à cette formule (II) et pouvant tout particulièrement être utilisés seuls ou sous forme d'un mélange de ces derniers, comme composé alcool dans le procédé selon l'invention.

**Tableau 1**

| Nom | R | Δ | Ch |
|---|---|---|---|
| 31-Norcycloartanol | Me | 9(19) | a |
| 31-Norcycloarténol | Me | 9(19) | b |
| Cycloeucalénol | Me | 9(19) | c |
| 31-Norcyclolaudénol | Me | 9(19) | d |
| 31-Norlanostérol | Me | 8(9) | a |
| 24(28)-Dihydroobtusifoliol | Me | 8(9) | e |
| Obtusifoliol | Me | 8(9) | c |
| 31-norparkéol | Me | 9(11) | b |
| 4a-méthylcrgosta-8,24-dièn-3pol | H | 8(9) | c |
| Lophénol | H | 7(8) | a |
| 4a-Méthylcholesta-7, 24-dièn-3β-ol | H | 7(8) | b |
| 24-Méthyllophénol (24R) | H | 7(8) | e |
| 24-Méthylènelophénol | H | 7(8) | c |
| 24-Ethyllophénol | H | 7(8) | f |
| Citrostadiénol | H | 7(8) | g |

(suite)

| Nom | R | Δ | Ch |
|---|---|---|---|
| Isocitrostadiénol | H | 7(8) | h |

Par "homologues hydrogénés" d'un 4-méthylstérol, on entend selon l'invention le ou les composé(s) 4-méthylstérol(s) correspondant(s) dont la ou les liaison(s) insaturée(s) éventuellement présente(s) ont été hydrogénées (c'est à dire transformée(s) en liaison saturée) selon des méthodes bien connues de l'homme du métier.

Parmi les alcools triterpéniques pouvant être avantageusement utilisés dans le procédé selon l'invention, on peut citer en particulier la β-amyrine, l'érythrodiol, le taraxastérol, le cycloarténol, le 24-méthylènecycloartanol, le lanostérol et les mélanges de ceux-ci.

Par "homologues hydrogénés" d'un alcool triterpénique, on entend selon l'invention le ou les composés alcool (s) triterpénique(s) correspondant(s) dont la ou les liaison(s) insaturée(s) éventuellement présente(s) ont été hydrogénées (c'est à dire transformée(s) en liaison saturée) selon des méthodes bien connues de l'homme du métier.

Les rapports molaires des réactifs pour le procédé selon l'invention sont tels que l'on utilise en particulier un rapport molaire corps gras : composé alcool compris entre environ 0,5 et environ 50, et plus particulièrement entre environ 1 et environ 2.

La température de la réaction d'estérification selon l'invention est de préférence comprise entre environ 100 et environ 400°C et plus particulièrement entre environ 200 et environ 250°C. La pression peut être comprise entre environ 0,05 et environ 50 bar et plus particulièrement entre environ 0,1 et environ 5 bar. Afin d'éviter une oxydation éventuelle des liaisons éthyléniques présentes, il est recommandé soit de purger le milieu réactionnel et les réactifs par un gaz inerte soit de réaliser la réaction sous un flux de gaz inerte ou un vide partiel, soit encore de réaliser la réaction sous une pression de gaz inerte comprise entre environ 0,1 et environ 5 bar.

Bien que le procédé selon l'invention peut avantageusement être mis en oeuvre en l'absence de solvant, on peut, le cas échéant, notamment lorsque l'alcool est difficilement soluble dans le corps gras, utiliser un solvant de préférence choisi dans le groupe constitué par les alcanes, les alcanes halogénés (notamment chlorés), le diméthylformamide (DMF) ou encore le diméthylsulfoxyde (DMSO).

Par le procédé selon l'invention, la réaction d'estérification conduit à l'obtention d'un ester présentant des qualités organoleptiques tout à fait acceptables (odeur, couleur, goût), un indice d'acide faible ainsi qu'une teneur minimale en alcool libre.

Bien entendu, le procédé selon l'invention peut comprendre en outre les étapes en amont ou en aval d'une réaction d'estérification catalysée connues de l'homme du métier.

En particulier, on peut améliorer encore la pureté du produit obtenu, par exemple par décoloration, désodorisation ou distillation moléculaire.

Par ailleurs, l'utilisation d'oxydes de lanthanide solides en tant que catalyseur solide permet la mise en oeuvre d'un procédé industriel plus respectueux de l'environnement (économie d'énergie, recyclage du catalyseur, des produits secondaires et des effluents et l'absence totale de solvants) dans la mesure où ces oxydes sont particulièrement faciles à récupérer et recyclables, par comparaison avec les catalyseurs d'estérification des stérols, stanols, les 4-méthylstérols et alcools triterpéniques déjà connus, qui nécessitent notamment leur destruction avec formation de sels.

Ainsi, par un choix approprié de la qualité pharmaceutique, notamment dermatologique, de la qualité cosmétique et/ou alimentaire des réactifs de départ, en utilisant les connaissances générales de l'homme du métier, et par la séparation améliorée du catalyseur du produit de réaction à la fin de l'estérification, le procédé selon l'invention permet d'obtenir des produits particulièrement adaptés à une utilisation dans les domaines de la pharmacie, notamment de la dermatologie, de la cosmétique et de l'alimentation particulière (aliments fonctionnels, alicaments, cosmetofood).

Ainsi, l'ester de corps gras tel qu'obtenu par le procédé d'estérification décrit ci-dessus peut être avantageusement utilisé en tant qu'agent pharmaceutiquement actif, notamment dermatologique, dans une composition pharmaceutique, notamment dermatologique.

En outre, l'ester de corps gras tel qu'obtenu par le procédé d'estérification décrit ci-dessus peut être avantageusement utilisé en tant qu'agent cosmétiquement actif dans une composition cosmétique.

Enfin, l'ester de corps gras tel qu'obtenu par le procédé d'estérification décrit ci-dessus peut-être avantageusement utilisé en tant qu'additif alimentaire.

Les exemples suivants sont destinés à illustrer la présente invention mais ne doivent en aucun cas être interprétés comme pouvant en restreindre la portée.

**Exemple 1 : utilisation de l'oxyde de lanthane pour la transestérification d'esters méthyliques avec un mélange de stérols**

**1) Caractéristiques des réactifs**

Le mélange de stérols utilisé est principalement composé de béta-sitostérol, à environ 50%. Une analyse détaillée de ce mélange, commercialisé par la société Henkel sous la dénomination "Generol" donne les résultats suivants (voir tableau 2) :

**Tableau 2**

| Produit | Composition |
|---|---|
| Campestérol | 26 à 31% |
| Campestanol | Traces |
| Stigmastérol | 16 à 23% |
| Bêta-sitostérol | 48 à 53% |
| Bêta-sitostanol | Traces |

Dans ce qui suit, ce mélange sera désigné par « stérols du mélange A».
Les esters méthyliques utilisés sont les suivants :

- le laurate de méthyle est d'origine industrielle, après analyse, sa pureté est d'au moins 98 % ;
- le myristate de méthyle et l'oléate de méthyle sont des produits industriels commerciaux dont les puretés respectives en oléate et en myristate sont de 87 % et 99 % en poids.

Dans chaque produit, le complément à 100 % est constitué par d'autres esters méthyliques de chaîne plus courte ou plus longue que celle des esters oléate ou myristate respectivement majoritaires.

2) Mode opératoire

La réaction de transestérification est mise en oeuvre à pression atmosphérique dans un réacteur quadricol en pyrex de 250 ml. Les caractéristiques du montage sont les suivantes:

- chauffage par l'intermédiaire d'un chauffe-ballon avec régulation de la température;
- agitation mécanique;
- arrivée d'azote;
- puits thermométrique permettant de mesurer la température à l'aide d'un thermocouple ;
- sortie du réacteur munie d'un réfrigérant afin de permettre l'élimination du méthanol formé, celui-ci qui est recueilli dans un tube collecteur.

On introduit dans le réacteur 29 g du mélange de stérols et une masse d'ester méthylique telle que l'on ait un rapport molaire des réactifs égal à 1 (soit 15 g de laurate de méthyle par exemple).
Les réactifs sont mis sous agitation à 500 trs/min et chauffés progressivement jusqu'à la température de 240 °C.
Une fois la température réactionnelle atteinte, au temps t=0, on prélève à la pipette Pasteur trois gouttes de mélange réactionnel. Ce prélèvement sera la référence pour l'ensemble des calculs.
Puis on introduit 2,316 g d'oxyde de lanthane $La_2O_3$ en tant que catalyseur solide, commercialisé par la société Rhodia sous la dénomination "HSA".
Ceci correspond donc à 5% en poids de catalyseur, par rapport au poids total du mélange réactionnel. L'oxyde de lanthane est sous forme d'une poudre de granulométrie telle que la taille moyenne des particules est d'environ 75 micromètres.
Enfin, on introduit de l'azote dans le montage, selon un débit de 80 ml/min, l'azote balayant la surface du mélange réactionnel et permettant d'entraîner le méthanol formé lors de la réaction de transestérification vers le tube collecteur.
Des prélèvements sont effectués au cours du temps, t=0, 0,5, 1, 2, 3, 5 et 7 heures. Ceux-ci sont pesés précisément, puis dissous dans 1 ml d'une solution d'héxadécane (étalon interne) dans le dodécane, à raison de 3,2

mmol d'étalon pour 50 ml de dodécane. Le dodécane, utilisé ici comme solvant, et l'héxadécane, l'étalon interne, sont respectivement des produits Sigma et Merck, leur pureté est de 99%. L'échantillon ainsi préparé est dosé par chromatographie en phase gazeuse au moyen d'un chromatographe VARIAN 3350 équipé d'un détecteur à ionisation de flamme et d'un injecteur on column. La séparation des produits est effectuée à l'aide d'une colonne capillaire SGE BP5.

La durée totale de la réaction est de 7heures.

**3) Exploitation des résultats**

**3.1 Définition et expression de la conversion**

Chaque prélèvement étant dissout dans 1 ml de solution d'hexadécane dilué dans du dodécane, on met donc en présence une quantité précise et constante d'hexadécane, soit $N_h$ = 6, 1. $10^{-5}$mol.

Ainsi, les analyses nous donnent les surfaces des produits, des réactifs, mais aussi celle de notre étalon. Connaissant les facteurs de réponse de chacune des espèces, facteurs déterminés par rapport à l'hexadécane, on peut donc aisément déterminer les quantités de réactifs et de produits présentes dans une masse $m_t$ d'un prélèvement effectué à un temps t.

Le premier prélèvement de masse $m_0$ nous permet de déterminer la quantité théorique d'un réactif A au temps t=0 :

$$Nth_{a0} = \frac{S_{a0} * N_b}{f_a * S_{h0}}$$

($Nth_{a0}$ et $N_b$ en mol)

$S_{a0}$ et $S_{h0}$ sont les surfaces du réactif A et de l'étalon relevées à l'issue de l'analyse du prélèvement effectué au temps t=0, $f_a$ est le facteur de réponse du réactif A.

De même, au temps t=t, la quantité de réactif A présente dans un prélèvement de masse $m_t$ est exprimée ainsi :

$$N_{at} = \frac{S_{at} * N_b}{f_a * S_{ht}}$$

Si le réactif A n'avait pas été consommé depuis le temps t=0, il y aurait en réalité une quantité théorique $Nth_{at}$ exprimée de la manière suivante :

$$Nth_{at} = \frac{Nth_{a0} * m_t}{m_0}$$

($Nth_{at}$ et $Nth_{a0}$ en mol, $m_0$ et m, en g)

On définit donc la conversion du réactif A comme étant le rapport de la quantité de réactif consommée sur la quantité de réactif théorique :

$$\%\ conversion\ A = 100\frac{Nth_{at} - N_{at}}{Nth_{at}}$$

**3.2 Définition et expression de la sélectivité d'un produit**

La sélectivité d'un produit est le rapport de la quantité de ce produit sur la somme des quantités des produits présents dans le prélèvement.

Dans le cas présent, comme il se forme des esters de stérols, des diènes et des sous-produits non identifiés

(que l'on surnomme ici "autres"), on obtient l'expression suivante :

$$\% \text{ sélectivité esters} = 100 \frac{N_{esters\,t}}{N_{esters\,t} + N_{diènes\,t} + N_{autres\,t}} \quad (\text{N en mol})$$

**3.3 Définition et expression du rendement de la réaction**

Dans le cas présent, le rendement de la réaction, exprimé en %, est le produit du % de conversion des stérols par le % de sélectivité en esters de stérols, le tout divisé par 100:

$$\text{Rendement } (\%) = (\% \text{ conversion stérols} * \% \text{ sélectivité esters}) / 100$$

4) Résultats

**4.1 Influence de la nature du catalyseur : transestérification du laurate de méthyle** par les stérols du mélange A

On utilise 5% en poids de chaque catalyseur, sous forme d'un poudre de granulométrie 75 micromètres. Le carbonate de potassium est utilisé comme catalyseur de référence.

<u>**Tableau 3**</u>

| Catalyseur (5 % en poids) | % conversion du laurate de méthyle | % conversion des stérols du mélange | rendement (%) |
|---|---|---|---|
| Reaction sans catalyseur | 25 | 74 | 38 |
| Oxyde de lanthane | 100 | 99 | 91 |
| Carbonate de potassium | 99 | 98 | 82 |

Par comparaison au carbonate de potassium, l'utilisation d'oxyde de lanthane selon l'invention permet d'obtenir un meilleur rendement en ester de stérol. Par ailleurs, en fin de réaction, le carbonate de potassium est pratiquement complètement dissous dans le milieu réactionnel, ce qui rend sa réutilisation quasiment impossible et son extraction du milieu réactionnel ardue (lavages successifs, etc.). Au contraire, l'oxyde de lanthane est directement séparable du milieu réactionnel par simple filtration.

**4.2 Influence de la surface spécifique du catalyseur : transestérification du laurate** de méthyle par les stérols du mélange A, catalysée par de l'oxyde de lanthane

<u>**Tableau 4**</u>

| Suaface spécifique de l'oxyde de lanthane ($m^2$/g) | % conversion du laurate de méthyle | % conversion des stérols du mélange | rendement (%) |
|---|---|---|---|
| 1 | 47 | 59 | 53 |
| 68 | 94 | 99 | 92 |

**4.3 Influence de la quantité de catalyseur : transestérification du laurate de méthyle par les stérols du mélange A**

**Tableau 5**

| Pourcentage massique | % conversion du laurate de méthyle (1) | % conversion des stérols du mélange | Rendement (%) |
|---|---|---|---|
| 5 | 47 | 59 | 53 |
| 10 | 81 | 88 | 81 |
| (1) Temps de réaction de 7 heures | | | |

**4.4 Influence de la température réactionnelle : transestérification du laurate de méthyle par les stérols du mélange A**

**Tableau 6**

| Température (°C) | % conversion du laurate de méthyle (1) | % conversion des stérols du mélange | rendement (%) |
|---|---|---|---|
| 220 | 86 | 92 | 87 |
| 230 | 97 | 96 | 90 |
| 240 | 100 | 99 | 91 |
| (1) Temps de réaction de 7 heures | | | |

**4.5 Influence de la nature de l'ester méthylique : transestérification de différents esters par les stérols du mélange A**

**Tableau 7**

| Ester | % conversion de l'ester | % conversion des stérols du mélange | rendement (%) |
|---|---|---|---|
| Laurate de méthyle | 100 | 99 | 91 |
| Myristate de 90 méthyle | | 99 | 87 |
| Oléate de méthyle | 100 | 99 | 86 |

**4.6 Transestérification des esters de l'huile de tournesol par les stérols du mélange A**

**Tableau 8**

| % conversion huile de tournesol | % conversion des stérols du mélange | rendement en esters de stérols (%) |
|---|---|---|
| 100 | 99 | 73 |

**Exemple 2 : comparaison avec le catalyseur solide oxyde de magnésium**

Une expérience est réalisée en présence de MgO dans les mêmes conditions opératoires que pour l'exemple 1 excepté le temps de réaction qui est de 5 heures. En particulier, l'oxyde de magnésium est sous forme d'une poudre de granulométrie similaire à celle de l'oxyde de lanthane qui est le même que celui utilisé à l'exemple 1. Les résultats sont reportés dans le tableau 9 suivant.

On constate qu'en présence d'oxyde de magnésium, l'ester formé à partir de laurate de méthyle et de béta-sitostérol est rapidement dégradé en d'autres produits. De plus, la forte basicité de l'oxyde de magnésium dopé par des éléments alcalins favorise la réaction de déshydratation du béta-sitostérol.

**Tableau 9**

| catalyseur solide | % Conv. Laurate | % Conv stérols | % Diènes | % esters | % autres | Rendement |
|---|---|---|---|---|---|---|
| $La_2O_3$ | 100 | 95 | 1 | 95 | 4 | 90 |
| MgO | 97.8 | 95.1 | 9 | 82 | 9 | 78 |
| Temps de réaction : 5 heures | | | | | | |

**Exemple 3 : capacité de recyclage du catalyseur solide oxyde de lanthane $La_2O_3$**

Dans un réacteur, on réalise quatre essais successifs identiques de réaction d'estérification, en présence du même catalyseur solide $La_2O_3$ que pour les exemples 1 et 2. A la fin de chaque essais, on sépare le catalyseur du milieu réactionnel par simple décantation et filtration puis on réintroduit dans le réacteur le catalyseur solide ainsi récupéré avec une nouvelle charge de réactifs (stérols et laurate de méthyle). Les résultats sont reportés dans le tableau 10 suivant, pour la première réaction et les trois autres suivantes désignées par "recyclage". On constate que les rendements ne sont pas modifiés de manière significative même après plusieurs cycles d'utilisation. Ces essais montrent clairement l'utilisation avantageuse du catalyseur solide oxyde de lanthane pour sa facilité de séparation à partir du produit de réaction et, qui plus est, sa capacité de recyclage.

**Tableau 10**

| Réaction | % Conv. Laurate | % Conv stérols | % Diènes | % esters | % autres | Rendement |
|---|---|---|---|---|---|---|
| 1ière réaction | 100 | 95 | 1 | 95 | 4 | 90 |
| 1er recyclage | 97 | 99 | 1 | 96 | 3 | 95 |
| 2ème recyclage | 96 | 99 | 1 | 94 | 5 | 93 |
| 3ème recyclage | 94 | 98 | 1 | 93 | 6 | 91 |
| Temps de réaction : 5 heures | | | | | | |

**Revendications**

1. Procédé de préparation d'un ester de corps gras, **caractérisé en ce que** l'on soumet à une réaction d'estérification au moins un corps gras avec au moins un composé alcool choisi dans le groupe constitué par les stérols, les stanols, les 4-méthylstérols et leurs homologues hydrogénés, les alcools triterpéniques et leurs homologues hydrogénés, et les mélanges de ceux-ci, en présence d'au moins un catalyseur solide choisi dans le groupe constitué par les oxydes de lanthanides et les mélanges de ces oxydes.

2. Procédé selon la revendication 1, **caractérisé en ce que** le catalyseur solide est choisi dans le groupe constitué par les oxydes de lanthane, les oxydes de cérium, les oxydes de praséodyme, les oxydes de néodyme, les oxydes de prométhium, les oxydes de samarium, les oxydes d'europium, les oxydes de gadolinium, les oxydes de terbium, les oxydes de dysprosium, les oxydes d'holmium, les oxydes d'erbium, les oxydes de thulium, les oxydes d'ytterbium, les oxydes de luténium et les mélanges de ces oxydes.

3. Procédé selon la revendication 1 ou 2, **caractérisé en ce que** le catalyseur solide est choisi dans le groupe constitué par l'oxyde de lanthane $La_2O_3$, l'oxyde cérique $CeO_2$, les oxydes de praséodyme $PrO_2$, $Pr_6O_{11}$ et $Pr_2O_3$, l'oxyde de samarium $Sm_2O_3$ et les mélanges de ces oxydes.

4. Procédé selon l'une quelconque des revendications précédentes, **caractérisé en ce que** le catalyseur solide est l'oxyde de lanthane $La_2O_3$.

5. Procédé selon l'une quelconque des revendications précédentes, **caractérisé en ce que** le catalyseur solide se présente sous une forme solide choisie dans le groupe constitué par les poudres, les grains (pellets), les billes, les formes extrudées et les mélanges de celles-ci.

6. Procédé selon l'une quelconque des revendications précédentes, **caractérisé en ce que** le catalyseur solide est

sous forme d'une poudre avec une taille moyenne de particules comprise entre environ 1 et environ 1000 micromètres.

**7.** Procédé selon l'une quelconque des revendications précédentes, **caractérisé en ce que** catalyseur solide est supporté sur un support inerte.

**8.** Procédé selon l'une quelconque des revendications précédentes, **caractérisé en ce que** le catalyseur solide est présent dans le milieu réactionnel en une proportion comprise entre environ 0,01 et environ 30 % en poids, par rapport au poids total du milieu réactionnel.

**9.** Procédé selon l'une quelconque des revendications précédentes, **caractérisé en ce que** le corps gras comprend au moins une chaîne hydrocarbonée linéaire en $C_7$-$C_{30}$, insaturée ou non insaturée, éventuellement substituée.

**10.** Procédé selon la revendication 9, **caractérisé en ce que** le corps gras comprend au moins une chaîne hydrocarbonée linéaire en $C_7$-$C_{30}$ éthyléniquement insaturée, comprenant au moins une insaturation éthylénique.

**11.** Procédé selon la revendication 9, **caractérisé en ce que** le corps gras comprend au moins une chaîne hydrocarbonée linéaire en $C_7$-$C_{30}$ éthyléniquement insaturée, comprenant au moins deux insaturations éthyléniques, conjuguées ou non conjuguées.

**12.** Procédé selon l'une quelconque des revendications 1 à 9, **caractérisé en ce que** le corps gras est un acide gras saturé choisi dans le groupe constitué par l'acide caprique, l'acide laurique, l'acide stéarique, l'acide myristique, l'acide palmitique et les mélanges de ceux-ci.

**13.** Procédé selon l'une quelconque des revendications 1 à 11, **caractérisé en ce que** le corps gras est un acide gras insaturé choisi dans le groupe constitué par l'acide undécylénique, l'acide oléique, l'acide ricinoléique, l'acide linoléique, les acides oméga-3 acide linolénique et ceux de formules :

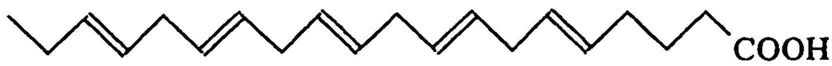

Acide Ecosopentaenoique (EPA, C20:5 / n-3)

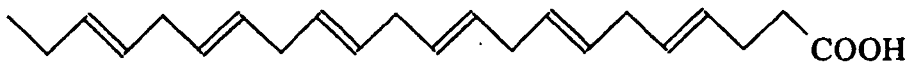

Acide Docosohexaenoique (DHA, C22:6 / n-3)

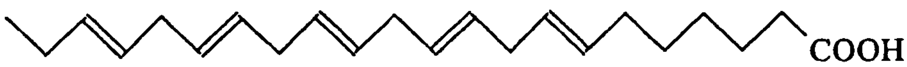

Acide Docosopentaenoique (DPA, C22:5 / n-3)

et les mélanges de ceux-ci.

**14.** Procédé selon l'une quelconque des revendications précédentes, **caractérisé en ce que** le corps gras est un acide gras d'au moins une huile végétale hydrogénée ou non.

**15.** Procédé selon l'une quelconque des revendications 1 à 11, **caractérisé en ce que** le corps gras est un ester d'un acide gras tel que les acides gras définis aux revendications 12 à 14.

**16.** Procédé selon la revendication 15, **caractérisé en ce que** le corps gras est un ester d'acide gras choisi dans le groupe constitué par le laurate de méthyle, le myristate de méthyle, l'oléate de méthyle, le linoléate de méthyle, le

stéarate de méthyle, l'undécylénate de méthyle, l'oléate de butyle, le ricinoléate de méthyle, le palmitate de méthyle, le palmitoélate de méthyle et les mélanges de ceux-ci.

**17.** Procédé selon l'une quelconque des revendications 1 à 11, **caractérisé en ce que** le corps gras est une huile végétale hydrogénée ou non ou un mélange d'huiles végétales hydrogénées ou non.

**18.** Procédé selon la revendication 14 ou 17, **caractérisé en ce que** l'huile végétale est choisie dans le groupe constitué par l'huile de tournesol, de palme, de palmiste, de noix de coco, de pépin de raisin, de moutarde noire, d'ocillette, de beurre de karité, d'amande douce, de soja, d'avocat, de lupin, d'arachide, de coton, de sésame, d'olive, de maïs, de cacao, de ricin, de Ben, de lin, de colza, de rocouyer, de germe de blé, de carthame, de noix, de noisette, de navette, l'huile de son de riz et les mélanges de celles-ci.

**19.** Procédé selon l'une quelconque des revendications précédentes, **caractérisé en ce que** le composé alcool est choisi dans le groupe des stérols et stanols répondant à la formule générale suivante :

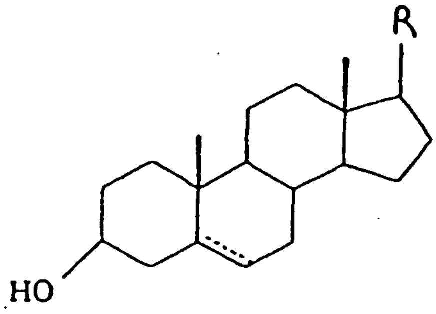

(I)

dans laquelle l'insaturation en pointillés en position 5 correspond à l'insaturation dans le cas des stérols, R représente une chaîne hydrocarbonée, linéaire ou ramifiée, insaturée ou non, comportant de 1 à 25 atomes de carbone. En particulier, R est choisi dans le groupe constitué par les groupes alkyle en $C_1$-$C_{12}$ les groupe alcoxy en $C_1$-$C_8$, les groupes alcényles en $C_2$-$C_8$, les groupe alcynyles en $C_2$-$C_8$, les groupes cycloalkyle en $C_3$-$C_8$, les groupes alcényles en $C_2$-$C_8$ halogénés, les groupes alcynyles en $C_2$-$C_8$ halogénés. Le terme "halogéné" désigne un ou plusieurs substituant halogène, à savoir un ou plusieurs atome(s) de chlore, fluor, brome ou iode.

**20.** Procédé selon la revendication 19, **caractérisé en ce que** le composé alcool est choisi dans le groupe constitué par le β-sitostérol, l'α-sitostérol, le γ-sitostérol, le stigmastérol, le campestérol, le β-sitostanol, le stigmastanol, le campestanol et les mélanges de ceux-ci.

**21.** Procédé selon l'une quelconque des revendications 1 à 18, **caractérisé en ce que** le composé alcool est un 4-méthylstérol choisi dans le groupe constitué par le 31-norcycloartànol, le 31-norcycloarténol, le cycloeucalénol, le 31-norcyclolaudénol, le 31-norlanostérol, le 24(28)-dihydioobtusifoliol, l'obtusifoliol, le 31-norparkéol, le 4a-méthylergosta-8, 24-dièn-3pol, le lophénol, le 4a-méthylcholesta-7, 24-dicn-3p-ol, le 24-méthyllophénol (24R), le 24-méthylènelophénol, le 24-éthyllophénol, le citrostadiénol, l'isocitrostadiénol et les mélanges de ceux-ci.

**22.** Procédé selon l'une quelconque des revendications 1 à 18, **caractérisé en ce que** le composé alcool est un alcool triterpénique choisi dans le groupe constitué par la β-amyrine, l'érythrodiol, le taraxastérol, le cycloarténol, le 24-méthylènecycloartanol, le lanostérol et les mélanges de ceux-ci.

**23.** Procédé selon l'une quelconque des revendications précédentes, **caractérisé en ce que** le rapport molaire corps gras : composé alcool est compris entre environ 0,5 et environ 50.

**24.** Procédé selon l'une quelconque des revendications précédentes, **caractérisé en ce que** la température de la réaction d'estérification selon l'invention est comprise entre environ 100 et environ 400°C.

**25.** Procédé selon l'une quelconque des revendications précédentes, **caractérisé en ce que** la pression est comprise entre environ 0,05 et environ 50 bar.

## Claims

1. A method for preparing a fatty substance ester, **characterized in that** at least one fatty substance with at least one alcohol compound selected from the group of sterols, stanols, 4-methyl-sterols and their hydrogenated homologues, triterpene alcohols and their hydrogenated homologues, and mixtures thereof, are submitted to an esterification reaction, in the presence of at least one solid catalyst selected from the group of lanthanide oxides and mixtures of these oxides.

2. The method according to claim 1,
**characterized in that** the solid catalyst is selected from the group of lanthanum oxides, cerium oxides, praseodymium oxides, neodymium oxides, promethium oxides, samarium oxides, europium oxides, gadolinium oxides, terbium oxides, dysprosium oxides, holmium oxides, erbium oxides, thulium oxides, ytterbium oxides, lutenium oxides and mixtures of these oxides.

3. The method according to claim 1 or 2,
**characterized in that** the solid catalyst is selected from the group of lanthanum oxide $La_2O_3$, ceric oxide $CeO_2$, praseodymium oxides $PrO_2$, $Pr_6O_{11}$ and $Pr_2O_3$, samarium oxide $Sm_2O_3$ and mixtures of these oxides.

4. The method according to any of the preceding claims, **characterized in that** the solid catalyst is lanthanum oxide $La_2O_3$.

5. The method according to any of the preceding claims, **characterized in that** the solid catalyst appears as a solid selected from the group of powders, grains (pellets), beads, extruded shapes and mixtures thereof.

6. The method according to any of the preceding claims, **characterized in that** the solid catalyst is in the form of a powder with an average particle size comprised between about 1 and about 1,000 micrometers.

7. The method according to any of the preceding claims, **characterized in that** the solid catalyst is supported on an inert support.

8. The method according to any of the preceding claims, **characterized in that** the solid catalyst is present in the reaction medium in a proportion comprised between about 0.01 and about 30% by weight, based on the total weight of the reaction medium.

9. The method according to any of the preceding claims, **characterized in that** the fatty substance comprises at least one unsaturated or non-unsaturated, optionally substituted, linear $C_7$-$C_{30}$ hydrocarbon chain.

10. The method according to claim 9,
**characterized in that** the fatty substance comprises at least one ethylenically unsaturated linear $C_7$-$C_{30}$ hydrocarbon chain comprising at least one ethylenic unsaturation.

11. The method according to claim 9,
**characterized in that** the fatty substance comprises at least one ethylenically unsaturated linear $C_7$-$C_{30}$ hydrocarbon chain comprising at least two conjugate or non-conjugate ethylenic unsaturations.

12. The method according to any claims 1 to 9,
**characterized in that** the fatty substance is a saturated fatty acid selected from the group of capric acid, lauric acid, stearic acid, myristic acid, palmitic acid and mixtures thereof.

13. The method according to any claims 1 to 11, **characterized in that** the fatty substance is an unsaturated fatty acid selected from the group of undecylenic acid, oleic acid, ricinoleic acid, linoleic acid, omega-3 acids, linolenic acid and those of formulae:

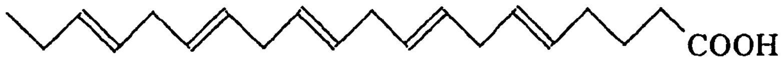

Eicosapentaenoic acid (EPA, C20:5 / n-3)

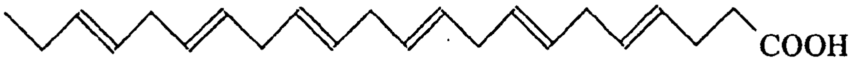

Docosahexaenoic acid (DHA, C22:6 / n-3)

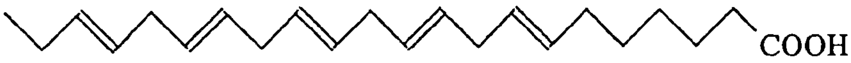

Docosapentaenoic acid (DPA, C22:5 / n-3)

and mixtures thereof.

**14.** The method according to any of the preceding claims, **characterized in that** the fatty substance is a fatty acid of at least one vegetable oil, either hydrogenated or not.

**15.** The method according to any claims 1 to 11, **characterized in that** the fatty substance is an ester of a fatty acid such as the fatty acids defined in claims 12 to 14.

**16.** The method according to claim 15,
**characterized in that** the fatty substance is a fatty acid ester selected from the group of methyl laurate, methyl myristate, methyl oleate, methyl linoleate, methyl stearate, methyl undecylenate, butyl oleate, methyl ricinoleate, methyl palmitate, methyl palmitoleate, and mixtures thereof.

**17.** The method according to any claims 1 to 11,
**characterized in that** the fatty substance is a vegetable oil, either hydrogenated or not, or a mixture of vegetable oils, either hydrogenated or not.

**18.** The method according to claim 14 or 17,
**characterized in that** the vegetable oil is selected from the group of sunflower oil, palm oil, palm kernel oil, coconut oil, grape seed oil, black mustard oil, poppy-seed oil, shea butter, sweet almond oil, soya bean oil, avocado oil, lupine oil, groundnut oil, cotton seed oil, sesame oil, olive oil, maize oil, cocoa oil, castor oil, Ben oil, flax oil, rapeseed oil, annatto oil, wheat germ oil, safflower oil, walnut oil, hazelnut oil, turnip seed oil, rice bran oil and mixtures thereof.

**19.** The method according to any of the preceding claims, **characterized in that** the alcohol compound is selected from the group of sterols and stanols of the following general formula:

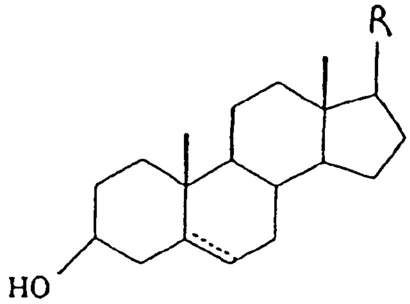

(I)

wherein the unsaturation in dotted lines in position 5 corresponds to unsaturation in the case of sterols, R represents an unsaturated or non-unsaturated linear or branched hydrocarbon chain, including 1 to 25 carbon atoms; in particular R is selected from $C_1$-$C_{12}$ alkyl groups, $C_1$-$C_8$ alkoxy groups, $C_2$-$C_8$ alkenyl groups, $C_2$-$C_8$ alkynyl groups, $C_3$-$C_8$ cycloalkyl groups, halogenated $C_2$-$C_8$ alkenyl groups, halogenated $C_2$-$C_8$ alkynyl groups; the term "halogenated" designates one or several halogen substituents, i.e. one or several chlorine, fluorine, bromine or iodine atom(s).

**20.** The method according to claim 19,
**characterized in that** the alcohol compound is selected from the group of β-sitosterol, α-sitosterol, γ-sitosterol, stigmasterol, campesterol, β-sitostanol, stigmastanol, campestanol, and mixtures thereof.

**21.** The method according to any claims 1 to 18, **characterized in that** the alcohol compound is a 4-methylsterol selected from the group of 31-norcycloartanol, 31-norcycloartenol, cycloeucalenol, 31-norcyclolaudenol, 31-norlanosterol, 24(28)-dihydroobtusifoliol, obtusifoliol, 31-norparkeol, 4α-methylergosta-8,24-dien-3βol, lophenol, 4α-methylcholesta-7,24-dien-3β-ol, 24-methyllophenol (24R), 24-methylene-lophenol, 24-ethyl-lophenol, citrostadienol, isocitrostadienol, and mixtures thereof.

**22.** The method according to any claims 1 to 18,
**characterized in that** the alcohol compound is a triterpene alcohol selected from the group of β-amyrin, erythrodiol, taraxasterol, cycloartenol, 24-methylene-cycloartanol, lanosterol and mixtures thereof.

**23.** The method according to any of the preceding claims, **characterized in that** the fatty substance/alcohol compound molar ratio is comprised between about 0.5 and about 50.

**24.** The method according to any of the preceding claims, **characterized in that** the temperature of the esterification reaction according to the invention is comprised between about 100 and about 400°C.

**25.** The method according to any of the preceding claims, **characterized in that** the pressure is between about 0.05 and about 50 bars.

**Patentansprüche**

**1.** Verfahren zur Herstellung eines Fettstoffesters, **dadurch gekennzeichnet, dass** mindestens ein Fettstoff mit mindestens einer Alkoholverbindung, die ausgewählt ist aus der Gruppe, die von den Sterolen, den Stanolen, den 4-Methylsterolen und ihren hydrogenierten Äquivalenten, den Triterpenalkoholen und ihren hydrogenierten Äquivalenten und den Gemischen derselben gebildet wird, in Anwesenheit mindestens eines festen Katalysators, der ausgewählt ist aus der Gruppe, die von den Lanthanidoxiden und den Gemischen dieser Oxide gebildet wird, einer Veresterungsreaktion unterzogen wird.

**2.** Verfahren nach Anspruch 1, **dadurch gekennzeichnet, dass** der feste Katalysator aus der Gruppe ausgewählt ist, die von den Lanthanoxiden, den Ceriumoxiden, den Praseodymoxiden, den Neodymoxiden, den Promethiumoxiden, den Samariumoxiden, den Europiumoxiden, den Gadoliniumoxiden, den Terbiumoxiden, den Dysprosiumoxiden, den Holmiumoxiden, den Erbiumoxiden, den Thuliumoxiden, den Ytterbiumoxiden, den Luteniumoxiden und den Gemischen dieser Oxide gebildet wird.

**3.** Verfahren nach Anspruch 1 oder 2, **dadurch gekennzeichnet, dass** der feste Katalysator aus der Gruppe ausgewählt ist, die vom Lanthanoxid $La_2O_3$, dem Cerioxid $CeO_2$, den Praseodymoxiden $PrO_2$, $Pr_6O_{11}$ und $Pr_2O_3$, dem Samariumoxid $Sm_2O_3$ und den Gemischen dieser Oxide gebildet wird.

**4.** Verfahren nach einem der vorangehenden Ansprüche, **dadurch gekennzeichnet, dass** der feste Katalysator das Lanthanoxid $La_2O_3$ ist.

**5.** Verfahren nach einem der vorangehenden Ansprüche, **dadurch gekennzeichnet, dass** sich der feste Katalysator in einer festen Form darstellt, die ausgewählt ist aus der Gruppe, die von den Pulvern, den Körnern (Pellets), den Kugeln, den extrudierten Formen und den Gemischen derselben gebildet wird.

**6.** Verfahren nach einem der vorangehenden Ansprüche, **dadurch gekennzeichnet, dass** der feste Katalysator in Form eines Pulvers ist mit einer durchschnittlichen Teilchengröße zwischen zirka 1 und zirka 1000 Mikrometer inklusive.

**7.** Verfahren nach einem der vorangehenden Ansprüche, **dadurch gekennzeichnet, dass** der feste Katalysator auf einem inerten Schlepper getragen wird.

**8.** Verfahren nach einem der vorangehenden Ansprüche, **dadurch gekennzeichnet, dass** der feste Katalysator im Reaktionsmilieu in einem Verhältnis zwischen zirka 0,01 und zirka 30 Gew.-% inklusive im Verhältnis zum Gesamtgewicht des Reaktionsmilieus vorhanden ist.

**9.** Verfahren nach einem der vorangehenden Ansprüche, **dadurch gekennzeichnet, dass** der Fettstoff mindestens eine ungesättigte oder nicht ungesättigte eventuell substituierte lineare $C_7$-$C_{30}$-Kohlenwasserstoffkette umfasst.

**10.** Verfahren nach Anspruch 9, **dadurch gekennzeichnet, dass** der Fettstoff mindestens eine ethylenisch ungesättigte lineare $C_7$-$C_{30}$-Kohlenwasserstoffkette umfasst, die mindestens einen ethylenisch ungesättigten Zustand umfasst.

**11.** Verfahren nach Anspruch 9, **dadurch gekennzeichnet, dass** der Fettstoff mindestens eine ethylenisch ungesättigte lineare $C_7$-$C_{30}$-Kohlenwasserstoffkette umfasst, die mindestens zwei konjugierte oder nicht konjugierte ethylenisch ungesättigte Zustände umfasst.

**12.** Verfahren nach einem der Ansprüche 1 bis 9, **dadurch gekennzeichnet, dass** der Fettstoff eine gesättigte Fettsäure ist, die aus der Gruppe ausgewählt ist, die von der Caprinsäure, der Laurinsäure, der Stearinsäure, der Myristinsäure, der Palmitinsäure und den Gemischen derselben gebildet wird.

**13.** Verfahren nach einem der Ansprüche 1 bis 11, **dadurch gekennzeichnet, dass** der Fettstoff eine ungesättigte Fettsäure ist, die aus der Gruppe ausgewählt ist, die von der Undecylensäure, der Ölsäure, der Rizinolsäure, der Linolsäure, der Omega-3-Säuren, der Linolensäure und den Säuren der Formeln:

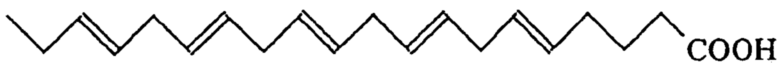

Eicosapentaensäure (EPA, C20:5 / n-3)

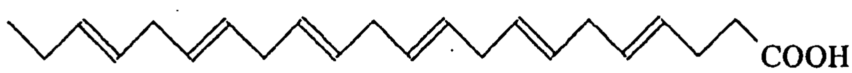

Docosahexaen-Säure (DHA, C22:6 / n-3)

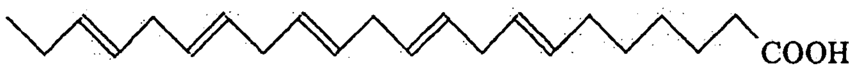

Docosapentaensäure (DPA, C22:5 / n-3)

und den Gemischen derselben gebildet wird.

**14.** Verfahren nach einem der vorangehenden Ansprüche, **dadurch gekennzeichnet, dass** der Fettstoff eine Fettsäure mindestens eines Pflanzenöls, hydrogeniert oder nicht, ist.

**15.** Verfahren nach einem der Ansprüche 1 bis 11, **dadurch gekennzeichnet, dass** der Fettstoff ein Ester einer Fettsäure wie der Fettsäuren ist, die in den Ansprüchen 12 bis 14 definiert sind.

**16.** Verfahren nach Anspruch 15, **dadurch gekennzeichnet, dass** der Fettstoff ein Fettsäureester ist, der aus der Gruppe ausgewählt ist, die von dem Laurylalkohol, dem Methylmyristat, dem Methyloleat, dem Methyllinoleat, dem Methylstearat, dem Methylundecylenat, dem Butyloleat, dem Methylricinoleat, dem Methylpalmitat, dem Methylpalmitoleat und den Gemischen derselben gebildet wird.

**17.** Verfahren nach einem der Ansprüche 1 bis 11, **dadurch gekennzeichnet, dass** der Fettstoff ein Pflanzenöl ist, hydrogeniert oder nicht, oder ein Gemisch von Pflanzenölen, hydrogeniert oder nicht.

**18.** Verfahren nach Anspruch 14 oder 17, **dadurch gekennzeichnet, dass** das Pflanzenöl aus der Gruppe ausgewählt ist, die von dem Sonnenblumen-, Palm-, Palmkern-, Kokosnuss-, Traubenkern-, Schwarzen Senf-, Mohn-, Sheabutter-, Süßmandel-, Soja-, Avocado-, Lupinen-, Erdnuss-, Baumwoll-, Sesam-, Oliven-, Mais-, Kakao-, Rizinus-, Behen-, Lein-, Raps-, Orlean-, Weizenkeim-, Saflor-, Walnuss-, Haselnuss-, Rübsen- und Reiskleienöl und den Gemischen derselben gebildet wird.

**19.** Verfahren nach einem der vorangehenden Ansprüche, **dadurch gekennzeichnet, dass** die Alkoholverbindung aus der Gruppe der Sterole und Stanole ausgewählt ist, die der folgenden allgemeinen Formel entsprechen:

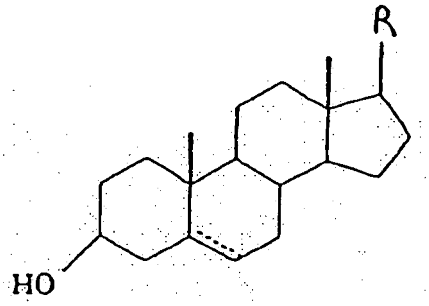

(I)

wobei der ungesättigte Zustand in Punktlinie an Position 5 dem ungesättigtem Zustand im Fall der Sterole entspricht, R eine lineare oder verzweigte Kohlenwasserstoffkette, ungesättigt oder nicht, darstellt, 1 bis 25 Kohlenstoffatome umfassend. Insbesondere ist R aus der Gruppe ausgewählt, die von den $C_1$-$C_{12}$-Alkylgruppen , den $C_1$-$C_8$-Alkoxygruppen, den $C_2$-$C_8$-Alkenylgruppen, den $C_2$-$C_8$-Alkynylgruppen, den $C_3$-$C_8$-Cycloalkylgruppen, den halogenierten $C_2$-$C_8$-Alkenylgruppen, den halogenierten $C_2$-$C_8$-Alkynylgruppen gebildet wird. Der Begriff "halogeniert" bezeichnet einen oder mehrere Halogen-Substituenten, nämlich ein oder mehrere Chlor-, Fluor-, Brom- oder Jodatom(e).

**20.** Verfahren nach Anspruch 19, **dadurch gekennzeichnet, dass** die Alkoholverbindung aus der Gruppe ausgewählt ist, die von dem β-Sitosterol, dem α-Sitosterol, dem γ-Sitosterol, dem Stigmasterol, dem Campesterol, dem β-Sitostanol, dem Stigmastanol, dem Campestanol und den Gemischen derselben gebildet wird.

**21.** Verfahren nach einem der Ansprüche 1 bis 18, **dadurch gekennzeichnet, dass** die Alkoholverbindung ein 4-Methylsterol ist, ausgewählt aus der Gruppe, die von dem 31-Norcycloartanol, dem 31-Norcycloartenol, dem Cycloeucalenol, dem 31-Norcyclolaudenol, dem 31-Norlanosterol, dem 24(28)-Dihydroobtusifoliol, dem Obtusifoliol, dem 31-Norparkeol, dem 4α-Methylergosta-8,24-dien-3βol, dem Lophenol, dem 4α-Methylcholesta-7,24-dien-3β-ol, dem 24-Methyllophenol (24R), dem 24-Methylenlophenol, dem 24-Ethyllophenol, dem Citrostadienol, dem Isocitrostadienol und den Gemischen derselben gebildet wird.

**22.** Verfahren nach einem der Ansprüche 1 bis 18, **dadurch gekennzeichnet, dass** die Alkoholverbindung ein Triterpenalkohol ist, der aus der Gruppe ausgewählt ist, die von dem β-Amyrin, dem Erythrodiol, dem Taraxasterol, dem Cycloartenol, dem 24-Methylencycloartanol, dem Lanosterol und den Gemischen derselben gebildet wird.

**23.** Verfahren nach einem der vorangehenden Ansprüche, **dadurch gekennzeichnet, dass** das molare Verhältnis Fettstoff : Alkoholverbindung zwischen zirka 0,5 und zirka 50 inbegriffen ist.

**24.** Verfahren nach einem der vorangehenden Ansprüche, **dadurch gekennzeichnet, dass** die Temperatur der erfindungsgemäße Veresterungsreaktion zwischen zirka 100 und zirka 400 °C inklusive ist.

**25.** Verfahren nach einem der vorangehenden Ansprüche, **dadurch gekennzeichnet, dass** der Druck zwischen zirka 0,05 und zirka 50 bar inbegriffen ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*


### Documents brevets cités dans la description

- US 5502045 A
- EP 585071 A

### Littérature non-brevet citée dans la description

- **LING ; JONES.** *Life Sciences,* 1995, vol. 57 (3), 195-206
- **DELPLANQUE.** *Oléagineux Corps Gras Lipides,* 1999, vol. 1, 86-93
- **WACHTER ; SALKA ; MAGNET.** *Cosmetics & Toileteries,* 1995, vol. 110, 72-80